# EUROPEAN PATENT SPECIFICATION

(11) **EP 3 811 889 B1**
(45) Date of publication and mention of the grant of the patent: **12.06.2024**
(21) Application number: 19205112.6
(22) Date of filing: 24.10.2019
(51) Int. Cl.: A61B 34/20, A61B 34/00

(54) **SURGERY ASSISTIVE SYSTEM FOR OBTAINING SURFACE INFORMATION**
OPERATIONSUNTERSTÜTZUNGSSYSTEM ZUM ERHALTEN VON OBERFLÄCHENINFORMATIONEN
SYSTÈME D'ASSISTANCE CHIRURGICALE POUR OBTENIR INFORMATIONS DE SURFACE

(43) Date of publication of application: 28.04.2021
(73) Proprietor: Point Robotics (Singapore) Pte. Ltd., Singapore 02-00 (SG)
(72) Inventor: CHOU, Hao-Kai, Hsinchu County 302 (TW); YANG, Chih-Min, Hsinchu County 302 (TW); YEN, Chia-Ho, Hsinchu County 302 (TW); YU, Shou-An, Hsinchu County 302 (TW); HUANG, Wei-Jhen, Hsinchu County 302 (TW); SU, Che-Wei, Hsinchu County 302 (TW); JUANG, Shyue-Cherng, Hsinchu County 302 (TW)
(74) Representative: Yang, Shu

(56) References cited:
- EP-A2- 1 879 499
- EP-A2- 3 360 502
- US-A1- 2018 235 715
- US-A1- 2018 263 714

## Description

### FIELD

The present disclosure relates to a surgery assistive system, and more particularly to a method for registration that enhances accuracy and precision of computer-assisted surgeries performed by the surgery assistive system.

### BACKGROUND

Numerous surgical operations require high manual precision on the part of the surgeon. For example, surgical orthopedic operations require the surgeon to mill, drill or saw a bone of a subject at a precise location and at a precise angle in order to fit a given implant into the bone or to shape the bone to create a desired geometric profile. Such operations are usually performed by free-hand, with the surgeon holding a specific surgical instrument and following a trajectory based on anatomical landmarks. Accuracy of the surgical operations is thus dependent on the skill of the surgeon in following the predetermined plan with the hand-held surgical instrument.

Taking the advantages of information technology and robotics, computer assisted surgery has offered a reliable option in improving the accuracy and precision of surgical operations. Computer assisted surgery represents a surgical concept that utilizes computer technology to visualize operating fields in a preoperative virtual environment to allow a more accurate preoperative diagnostic and well-defined surgical planning. In computer assisted surgeries, patient registration is a critical preoperative procedure that correlates positions of a virtual three-dimensional (3D) dataset gathered by computer medical imaging, such as computed tomography (CT) or magnetic resonance imaging (MRI), with positions of the patient. Patient registration eliminates the necessity of maintaining the patient in the same strict position during both preoperative scanning and surgery, and ensures geometrical accuracy of surgical operations.

Conventional registration method primarily includes fiducial registration and surface matching registration. Fiducial registration registers a specific anatomical region to the computer assisted navigation system by detecting a plurality of fiducial markers that are attached onto anatomical landmarks to define the anatomical region. Surface matching registration is performed by utilizing mechanical or ultrasound probes to identify the coordinates of a set of points on an anatomical surface structure of an operating field, thus offering higher accuracy in spatial recognition and reduced surgical invasiveness over fiducial registration.

In conventional surface matching registration, the probe may be coupled to a sensor to detect the mechanical contact of the probe with the anatomical surface. For example, Shen et al. discloses in U.S. Patent No. 8615286 a device and method for finding the location of a bone surface in patients that utilizes a force sensor installed at the base of a thin probe to detect resistance of the material encountered by the probe to discriminate engagement of the probe with bone or soft tissues or with different types of tissue with dissimilar hardness.

However, in actual surgical operations, the conventional thin probe tends to slip off the intended surface of contact due to the lack of control over the precise contact angle of the probe or the varying hardness between different layers of tissues, therefore resulting in significant reduction in accuracy of the registration. None of the existing art provides a mean that can control the angle of contact of the probe on the anatomical surface or determine the validity of the mechanical contact between the probe and the surface. EP 3360502 A2 discloses a surgical system with a robotic arm and a force sensor which can be used for registration purposes. The user is prompted to touch fiducials with the tool.

EP1879499 A2 discloses robotic surgical systems with built-in navigation capability for patient position tracking and surgical instrument guidance during a surgical procedure, without the need for a separate navigation system. Robotic based navigation of surgical instruments during surgical procedures allows for easy registration and operative volume identification and tracking. The systems, apparatus, and methods herein allow re-registration, model updates, and operative volumes to be performed intra-operatively with minimal disruption to the surgical workflow. In certain embodiments, navigational assistance can be provided to a surgeon by displaying a surgical instrument's position relative to a patient's anatomy. Additionally, by revising pre-operatively defined data such as operative volumes, patient-robot orientation relationships, and anatomical models of the patient, a higher degree of precision and lower risk of complications and serious medical error can be achieved.

### BRIEF SUMMARY OF THE DISCLOSURE

An objective of the present disclosure is to provide a surgical instrument that controls and detects the angle of contact of a registration probe thereof on an anatomical surface.

Another objective of the present disclosure is to provide a registration method for the surgical instrument that validates the mechanical contact between the registration probe and the anatomical surface.

The invention provides a surgery assistive system in accordance with independent claim 1.

### BRIEF DESCRIPTION OF THE DRAWINGS

The accompanying drawings illustrate one or more embodiments of the present disclosure and, together with the written description, explain the principles of the present disclosure. Wherever possible, the same reference numbers are used throughout the drawings to refer to the same or like elements of an embodiment.
FIG. 1 is a block diagram of a surgery assistive system in accordance with an embodiment of the present disclosure;
FIG. 2 is a schematic illustration of the surgery assistive system in accordance with an embodiment of the present disclosure;
FIG. 3 is a perspective view of a hand-held instrument of the surgery assistive system in accordance with an embodiment of the present disclosure;
FIG. 4 is a side view of the hand-held instrument of the surgery assistive system in accordance with an embodiment of the present disclosure;
FIG. 5 is a side view of a manipulator of the hand-held instrument of the surgery assistive system in accordance with an embodiment of the present disclosure;
FIG. 6 is a side view of another manipulator of the hand-held instrument of the surgery assistive system in accordance with an embodiment of the present disclosure;
FIG. 7 is a schematic illustration of an operation state of the surgery assistive system in accordance with an embodiment of the present disclosure;
FIG. 8 is a schematic illustration of an operation state of a spatial sensor system of the surgery assistive system in accordance with an embodiment of the present disclosure;
FIG. 9 is a flow diagram of an operation method of the surgery assistive system in accordance with a comparative example to the present disclosure;
FIG. 10 is a schematic illustration of a snapshot of a registration process of the surgery assistive system in accordance with an embodiment of the present disclosure;
FIG. 11 is a flow diagram of the steps of obtaining surface information for registration of the surgery assistive system in accordance with a comparative example to the present disclosure;
FIG. 12 is a schematic illustration of a target region and reference points generated by the surgery assistive system in accordance with an embodiment of the present disclosure;
FIG. 13 is a schematic illustration of a snapshot of the registration process of the surgery assistive system in accordance with an embodiment of the present disclosure;
FIG. 14 is a flow diagram of the steps of obtaining surface information for registration of the surgery assistive system in accordance with a comparative example to the present disclosure;
FIG. 15 is a flow diagram of the steps of validating sampling information for registration of the surgery assistive system in accordance with a comparative example to the present disclosure; and
FIG. 16 is an example of filtering sampling information to obtain surface information for registration of the surgery assistive system in accordance with an embodiment of the present disclosure.

In accordance with common practice, the various described features are not drawn to scale and are drawn to emphasize features relevant to the present disclosure. Like reference characters denote like elements throughout the figures and text.

### DETAILED DESCRIPTION OF THE PREFERRED EMBODIMENTS

The present disclosure will now be described more fully hereinafter with reference to the accompanying drawings illustrating various exemplary embodiments of the disclosure. The present disclosure may, however, be embodied in many different forms and should not be construed as limited to the embodiments set forth herein. Rather, these embodiments are provided so that this disclosure will be thorough and complete, and will fully convey the scope of the disclosure to those skilled in the art. Like reference numerals refer to like elements throughout.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising," or "includes" and/or "including" or "has" and/or "having" when used herein, specify the presence of stated features, regions, integers, steps, operations, elements, and/or components, but do not preclude the presence or addition of one or more other features, regions, integers, steps, operations, elements, components, and/or groups thereof.

It will be understood that the terms "and/or" and "at least one" include any and all combinations of one or more of the associated listed items. It will also be understood that, although the terms first, second, third etc. may be used herein to describe various elements, components, regions, parts and/or sections, these elements, components, regions, parts and/or sections should not be limited by these terms. These terms are only used to distinguish one element, component, region, part or section from another element, component, region, layer or section. Thus, a first element, component, region, part or section discussed below could be termed a second element, component, region, layer or section without departing from the teachings of the present disclosure.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

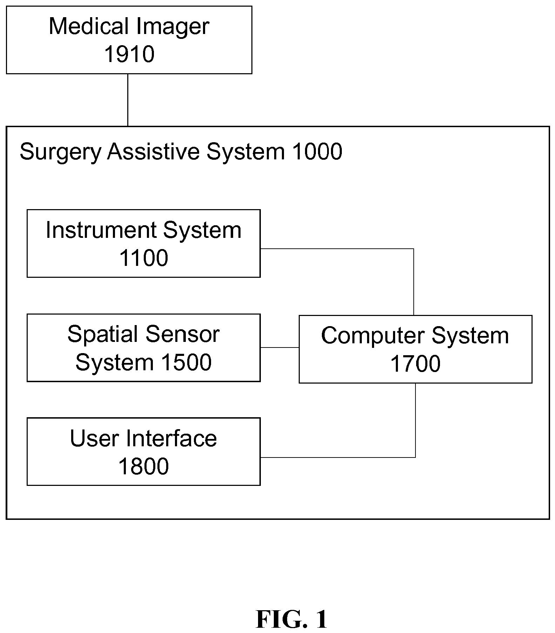

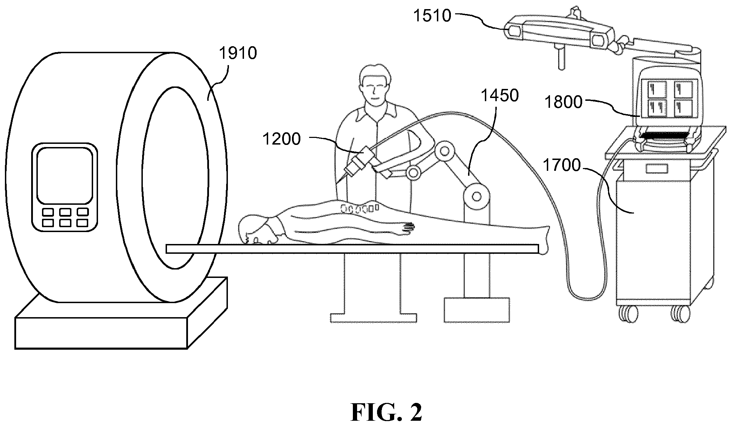

Referring now to FIG. 1 and FIG. 2. According to an embodiment of the present disclosure, a surgery assistive system 1000 for pre-surgical and surgical operations includes surgical hardware coupled with electronic modules and processor-executable instructions. The surgery assistive system 1000 includes an instrument system 1100, a spatial sensor system 1500, a user interface 1800, and a computer system 1700 electrically connected to the instrument system 1100, the spatial sensor system 1500, and the user interface 1800. In the embodiment, the surgery assistive system 1000 allows a user (e.g., a surgeon) to conduct surgery on a subject (e.g., a patient) by the instrument system 1100 with reference to the user interface 1800. At least one medical imager 1910 is in communication with the surgery assistive system 1000 and is configured to acquire medical images of the subject and transmit the images to the surgery assistive system 1000. The spatial sensor system 1500 is configured to generate spatial information of the subject and the environment. The computer system 1700 is configured to generate a virtual anatomical model according to the medical images and a surgical plan according to the virtual anatomical model, to track the surgical environment according to the spatial information received from the spatial sensor system 1500, and to control movement or alter the kinematic state of the manipulator 1210. The user interface 1800 visualizes the anatomical model and allows the user to navigate through the operating field according to the surgical plan.

As illustrated in FIG. 2, the instrument system 1100 of the surgery assistive system 1000 includes a hand-held instrument 1200 for performing surgery on the subject. In the embodiment, the instrument system 1100 may further include a support arm 1450 connected to the instrument 1200 to reduce weight load on the hands of the user and optionally provide more operational stability during surgeries.

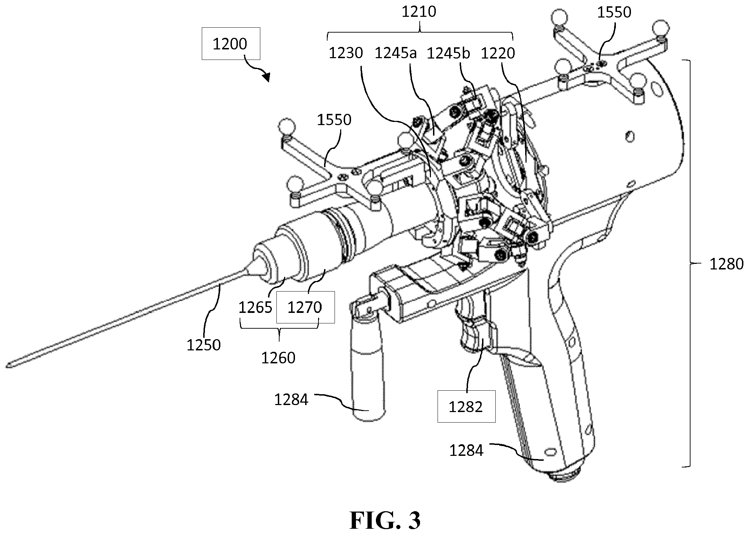

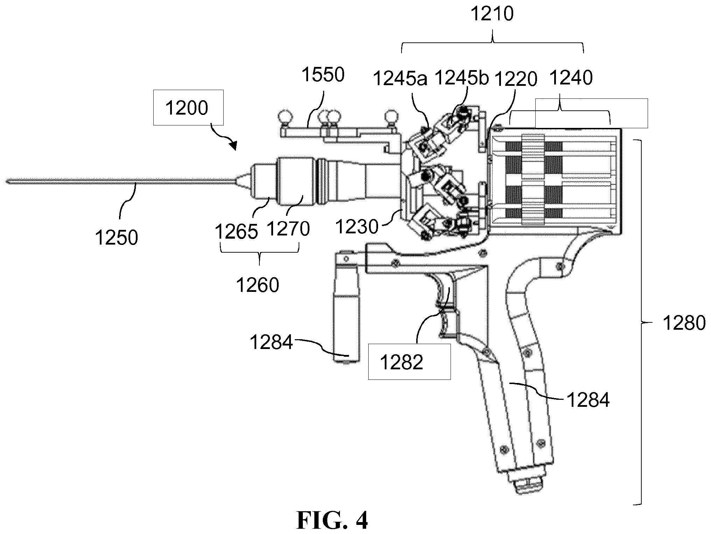

Referring to FIG. 3 and FIG. 4. According to an embodiment, the hand-held instrument 1200 includes a tool 1250, a tool installation base 1260, a manipulator 1210, and an instrument housing 1280. The tool 1250 is configured to contact or modify an anatomical surface on a body part of the subject. The tool installation base 1260 is connected to an end of the tool 1250 and the manipulator 1210 so that the tool 1250 is stably connected to the manipulator 1210. The manipulator 1210 is a mechanism controlled by the computer system 1700 for manipulating the position and orientation of the tool 1250. The instrument housing 1280 is connected to the manipulator 1210 to accommodate at least a portion of the manipulator 1210 and provide one or more handles 1284 for allowing the user to hold onto and maneuver the instrument 1200 during operation of the surgery assistive system.

In the embodiment, the tool 1250 may be a probe or indicator for contacting or assessing an anatomical site of the subject and detecting the structure or status of the anatomical site. The tool 1250 may be a drill bit, bur, curette, saw, screwdriver or other tool commonly used in surgical medicine that modifies or removes a portion of the tissues at the anatomical site by drilling, milling, cutting or scraping. In some embodiments, the tool 1250 is a mechanical, optical or ultrasound probe for performing surface matching registration and may be, but is not limited to, a rigid probe, a pressure sensor, a piezoelectric sensor, an elastomeric sensor, an optical camera, a laser scanner or an ultrasonic scanner.

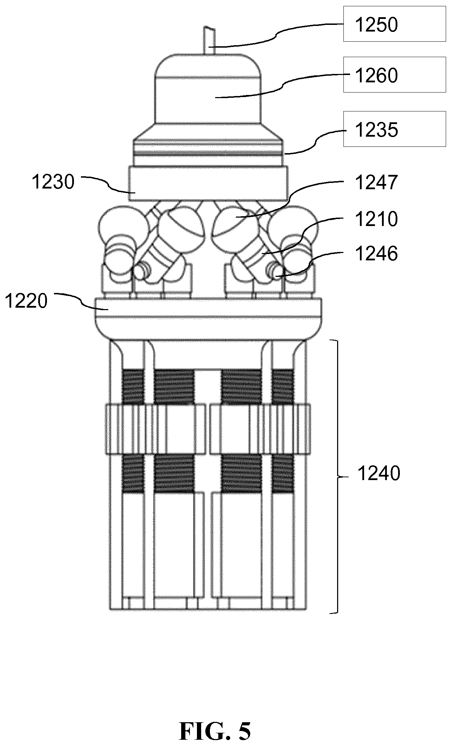

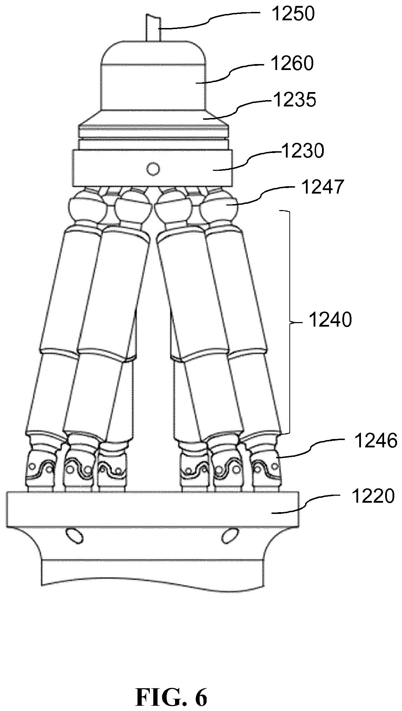

In the embodiment, the tool installation base 1260 is connected to the tool 1250 and a first side of a robotically controlled platform 1230 of the manipulator 1210. The tool installation base 1260 includes a tool adaptor 1265 and a motor 1270 connected to the tool adaptor 1265. The tool adaptor 1265 may be a clamp or other fastening structure for holding an end of the tool 1250 firmly to avoid displacement of the tool during operations. The motor 1270 may be a direct current (DC) motor or an alternating current (AC) motor for transducing electric energy into mechanical energy and generating a linear or rotary force to drive movement of the tool 1250. In an alternative embodiment, the motor may be disposed at the rear end of the instrument to reduce loading on the manipulator 1210 during operation of the instrument and to redistribute the weight of the instrument 1200 for improved user ergonomics. Additionally, as illustrated in FIG. 5 and FIG. 6, the tool installation base 1260 may further include a force sensor 1235 connected to the first side of the platform 1230 for detecting the force and/or torque sustained by the tool 1250 during surgeries. In other embodiments, the force sensor 1235 may be disposed in the probe or tool of the instrument; alternatively, the instrument 1200 may further include another force sensor (not shown in figures) disposed in the probe or tool. The force sensor may be, but is not limited to, a strain gauge, a force-sensitive resistor, a pressure transducer, a piezoelectric sensor, an electroactive polymer or an optical fiber bending sensor.

In the embodiment, the manipulator 1210 includes a base 1220, the platform 1230 connected to the tool installation base 1260, a plurality of joints 1245a, 1245b mounted on a second side of the platform 1230 away from the tool 1250 and on a first side of the base 1220 facing the platform 1230, and a plurality of actuators 1240 connected to the base 1220 on a second side of the base 1220 away from the platform 1230. As illustrated in FIG. 4, the base 1220 may be immobilized on or accommodated in the instrument housing 1280. The manipulator 1210 may be a parallel manipulator, such as a Stewart manipulator with six degrees of freedom (DOFs), for higher space efficiency and maneuverability. Additionally, the manipulator is preferably made of stainless steel or carbon fiber and arranged in a specific mechanical structure that allows the manipulator 1210 to possess sufficient sustainability against the force and/or torque generated from the tool 1250 contacting the subject during surgeries.

In the embodiment, the joints of the manipulator 1210 may be, but are not limited to, revolute joints, prismatic joints, spherical joints, universal joints, cylinder joint, or any combination thereof that enables a desired DOF. As exemplified in FIG. 5 and FIG. 6, the manipulator 1210 having a general Stewart platform with six DOFs may include universal joints 1246 and spherical joints 1247 to enable broad ranges of motion and various kinematic states of the manipulator 1210. The manipulator 1210 may further include a plurality of connectors, each being connected to one of the joints 1245a and one of the joints 1245b, to enable a broader range of movement of the tool 1250.

In the embodiment, the actuators 1240 of the manipulator 1210 connected to the base 1220 on the side opposite to the joints are configured to drive the joints, and the connectors if any, to move according to control signals transmitted from the computer system 1700. In an alternative embodiment, the actuators 1240 and the joints may be disposed on the same side of the base 1220. As exemplified in FIG. 6, the actuators 1240 are disposed between the base 1220 and the platform 1230, with each of the actuators 1240 being joined by a universal joint 1246 and a spherical joint 1247. The plurality of actuators 1240 may be linear actuators for higher precision and stronger sustainability.

Referring again to FIG. 3 and FIG. 4. In the embodiment, in addition to accommodating the manipulator 1210 and providing handles, the instrument housing 1280 may further include a control module 1282 for allowing the user to trigger, halt, or adjust actions of the tool 1250 or perform other functions of the instrument 1200.

In the embodiment, the hand-held instrument 1200 may be used with a calibration device 1300 configured to calibrate kinematic state of the manipulator 1210 in respect of the instrument housing 1280 so as to ensure geometric accuracy of the instrument 1200.

In the embodiment, the instrument 1200 may include at least one inertial measurement unit that detects acceleration, velocity, displacement, angular velocity and/or angular acceleration of the instrument 1200.

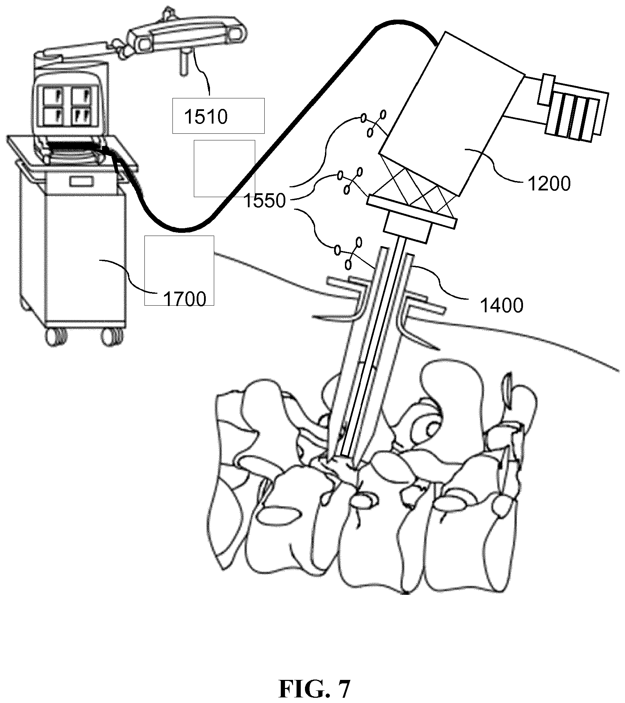

Referring to FIG. 7. The instrument 1200 may be used with a trocar 1400, especially in a minimally invasive surgery, to provide a physical portal for the tool 1250 of the instrument 1200 to reach the anatomical site of interest. In an alternative embodiment, the trocar 1400 may be removably connected to the platform 1230 of the manipulator 1210 to enable simultaneous entry of the trocar 1400 and the tool 1250 into the anatomical site.

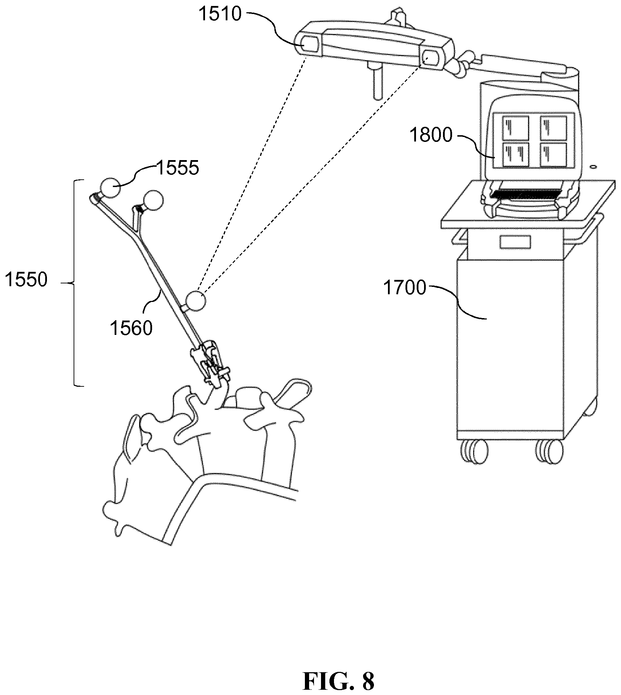

Referring now to FIG. 8. According to an embodiment of the present disclosure, the spatial sensor system 1500 of the surgery assistive system 1000 is configured to detect and thus enable tracking of the spatial information (e.g., location and orientation) of at least one target object, and includes at least one spatial marker frame 1550 removably attached to the target object, and a spatial sensor 1510 having at least one camera for receiving signals transmitted from the spatial marker frame 1550.

As exemplified in FIG. 7 and FIG. 8, the target object may be the instrument 1200, the trocar 1400, or a selected anatomical site. In the embodiment, the spatial marker frame 1550 includes a plurality of markers 1555 for emitting electromagnetic signals, sound wave, heat, or other perceivable signals, and an adaptor 1560 removably attached to the target object for holding the markers 1555 so that the target object becomes trackable by the spatial sensor 1510. In another embodiment, the spatial sensor system 1500 may further include a signal generator (not shown in figure) disposed on the spatial sensor 1510 or at a predefined location. Consequently, signal transmission by the markers 1555 may be active or passive; in other words, the signals emitted by the markers 1555 may be generated by the marker spheres, or the markers 1555 may be covered with reflective material so that signals generated by the signal generator are reflected by the markers 1555 to the spatial sensor 1510.

In the embodiment, the signal received by the spatial sensor 1510 is transmitted to the computer system 1700 and transformed into a coordinate system of the detected space and spatial information of the target object by triangulation or other transformation algorithm. Further, the markers 1555 of the spatial marker frame 1550 may be arranged on the adaptor 1560 in a specific pattern, as exemplified in FIG. 8, thus allowing the computer system 1770 to generate orientation information of the target object accordingly. The computer system 1700 may generate control signals according to the spatial and orientation information to control movement or alter kinematic state of the manipulator 1210 of the instrument 1200 or generate instructions to be shown on the user interface 1800 to prompt the user to move the instrument 1200 to a designated location or orientation.

According to an embodiment of the present disclosure, the computer system 1700 of the surgery assistive system 1000 includes a processor and a storage unit. The processor may be a general purpose processor, an application-specific instruction set processor or an application-specific integrated circuits that performs operations on a data source, such as the storage unit or other data stream. For example, the processor is an ARM based processor or an 8086x processor. In some embodiments, the processor further includes a plurality of digital or analog input/output, and may be a real-time operating system (RTOS) processor. The storage unit may store digital data assigned by the processor for immediate use in the computer system. The storage unit may be volatile, such as flash memory, read-only memory (ROM), programmable read-only memory (PROM), and erasable programmable read-only memory (EPROM), or non-volatile, such as dynamic random access memory (DRAM) and static random access memory (SRAM).

According to an embodiment, the user interface 1800 includes at least one output device for presenting information to the user and at least one input device. The information presented by the user interface 1800 may include, but is not limited to, surgical plans, two-dimensional (2D) or 3D reconstruction images, 2D or 3D drilling status (e.g., position, angle, depth or bending of the tool), compensation range of the tool, user guidance, warning area, notification of tool deviation from the surgical plan and notification of force sustainability limit of the tool. The output device may be a display, a light indicator or other visual means; alternatively, the output device may also be, or further include, a speech synthesizer or other audio means. The input device is capable of transducing commands entered by the user into electrical signals, and may be a pedal, a keyboard, a mouse, a touch panel, a voice recognition interface, or a gesture recognition interface.

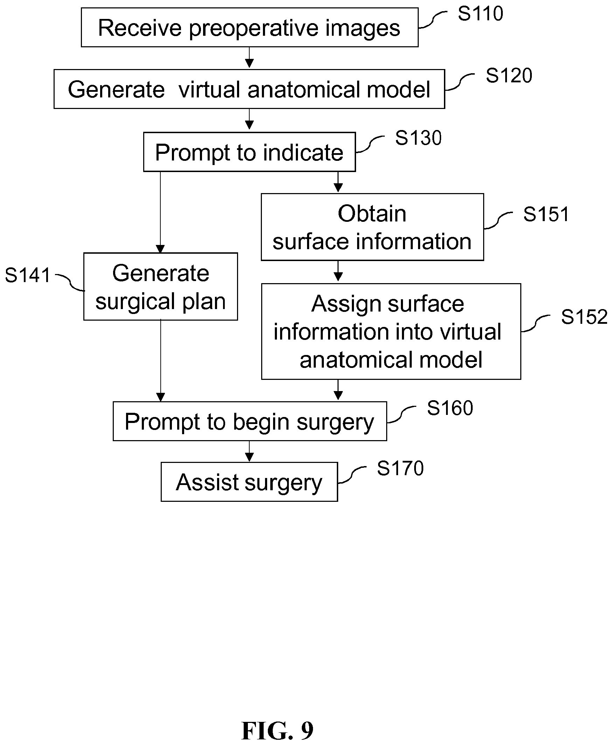

Referring to FIG. 9. According to a comparative example to the present disclosure, a method of performing a computer-assisted surgery by the surgery assistive system 1000 includes the steps of: (S110) receiving a plurality of medical images from the medical imager 1910; (S120) generating a three-dimensional virtual anatomical model according to the medical images; (S130) prompting the user to indicate location(s) of interest on the virtual anatomical model; (S141) generating a surgical plan according to the virtual anatomical model, the indicated location(s), and physiological and/or pathological information obtained from the medical images; (S160) prompting the user to begin surgery according to the surgical plan; and (S170) assisting the user during the surgery.

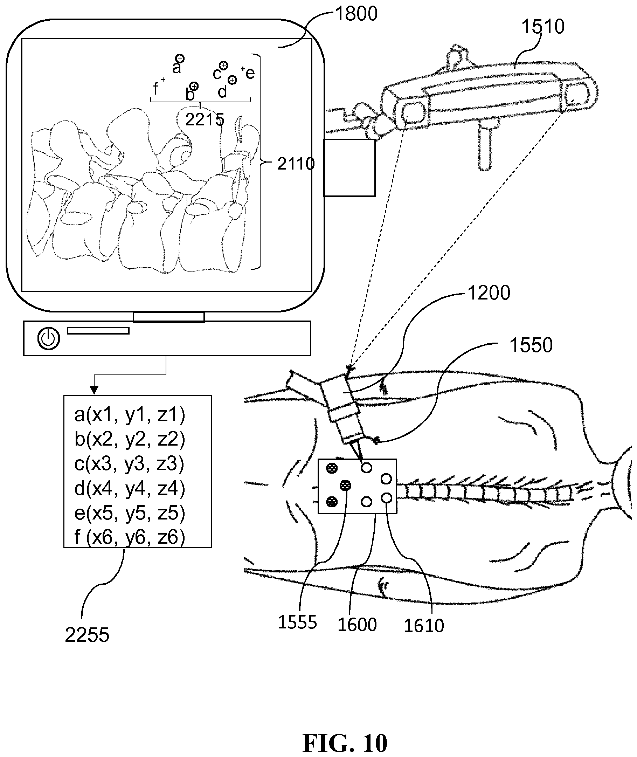

In Step S110, the medical imager 1910 may be a computed tomography (CT) scanner, a magnetic resonance imaging (MRI) scanner, or other commonly used medical imaging equipment that is capable of acquiring consecutive cross-sectional images of the scanned subject. In a preferred embodiment, a marker patch 1600, as illustrated in FIG. 10, is attached to the subject near the intended surgical site when the medical images are taken, to facilitate positioning of the surgical environment. Specifically, the marker patch 1600 may include at least one marker 1555 detectable by the spatial sensor 1510 of the spatial sensor system and a plurality of fiducial markers 1610 that cause markings on images taken by the medical imager 1910. The fiducial markers 1610 may be made of lead, iron, calcium, or other radiopaque metals. Therefore, as exemplified in FIG. 10, in the case where the marker patch 1600 is attached to the subject when taking the medical images, the resulting virtual anatomical model 2110 would include a plurality of radiopaque spots corresponding to the fiducial marker 1610.

In another embodiment, the markers 1555 on the marker patch 1600 may be disposed concentrically with the fiducial markers 1610 so as to avoid signal inconsistency caused by varying surface contour of the subject. Alternatively, the marker patch 1600 may be disposed with materials that are both optically readable by the spatial sensor system 1510 and radiopaque to the medical imager 1910 to ensure higher consistency between the acquired signals.

Referring again to FIG. 9. In Step S130, the user is prompted to indicate one or more locations of interest on the virtual anatomical model 2110 via the user interface 1800. The location of interest may include an intended surgical site or a specific anatomical landmark or surface feature. The user may also be allowed to label or define specific landmarks or surface features on the virtual anatomical model. In Step S141, the surgical plan generated by the method may include operative details, such as location and angle of tool entry and depth and path for the planned drilling, suggested type of tool, and suggested type of screw.

In Step 160, after the surgical plan is generated, the computer system 1700 prompts the user to begin surgery according to the surgical plan. The user may be allowed to adjust or edit the surgical plan before the surgery begins. In Step S170, the surgery assistive system 1000 assists the user during the planned surgery by adjusting the kinematic state of the manipulator 1210 according to the spatial information of the tool as detected by the spatial sensor system 1500, and informs the user via the user interface 1800. Further, in some embodiments, medical images may also be taken during the surgery to monitor the location, angle, and depth of the drilled path so as to ensure compliance with the surgical plan and to help determine the necessity to redefine a new surgical plan or to recalibrate the instrument.

After the user selects a location of his/her interest in the virtual anatomical model in Step S130, the method according to the embodiment may further include the steps of: (S151) obtaining surface information of a plurality of sampling points on the anatomical site of the subject; and (S152) assigning the surface information into the virtual anatomical model, thereby registering the virtual anatomical model into the coordinate system established by referencing the spatial information obtained by the spatial sensor system 1500.

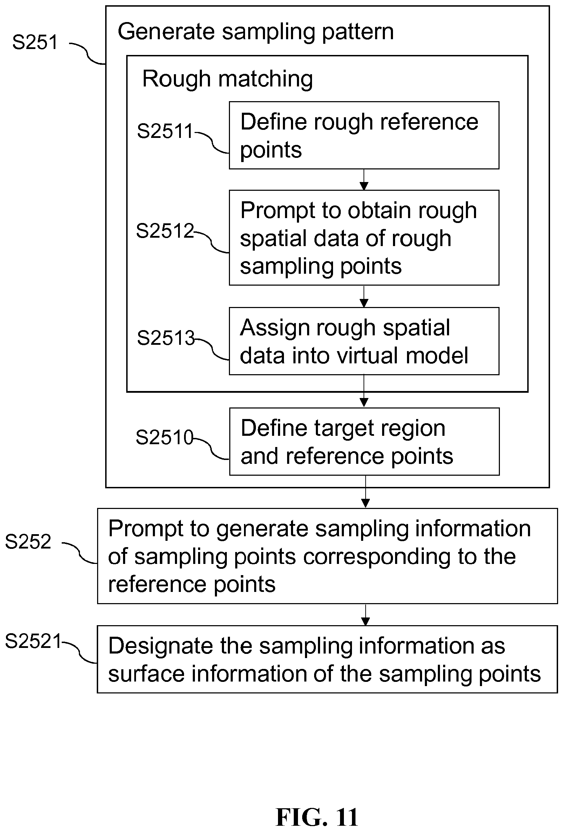

More specifically, as shown in FIG. 11, the step S151 of obtaining surface information of the sampling points according to the embodiment includes the steps of (S251) generating sampling pattern; (S252) prompting the user to generate sampling information of the sampling points; and (S2521) designating the sampling information as surface information of the sampling points.

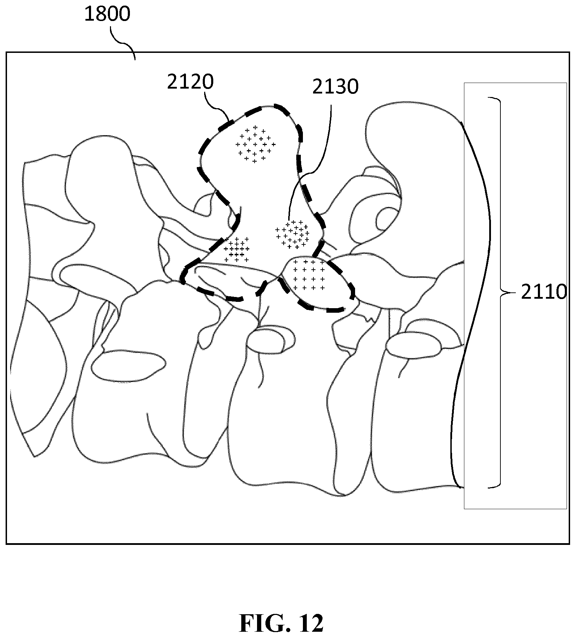

In the embodiment, the step S251 of generating sampling pattern includes (S2510) defining a target region and a plurality of reference points on the virtual anatomical model. As illustrated in FIG. 12, after the user indicates a location of interest on the virtual anatomical model 2110 via the user interface 1800, the computer system 1700 may define a target region 2120 and a plurality of reference points 2130 in the target region. Preferably, the target region 2120 is a region having characteristic or representative surface features (e.g., facet or spinous process of a spine) or a trocar accessible region. In at least one embodiment, the references points 2130 are so distributed in target region 2120 that a plurality of surface features are covered by the reference points 2130. Preferably, the amount of the reference points 2130 may be equal to or larger than 50. The reference points 2130 may be arranged orthogonally, concentrically, spirally, or in other patterns that preferably cover a significant number of surface features. In some embodiments, targeted precision of the reference points 2130 (i.e., the distance between two adjacent reference points 2130) may be set as 3 to 4 times of the resolution of the spatial sensor 1510; for example, when the spatial sensor 1510 has a resolution of 0.25 mm, the distance between two adjacent references points may be defaulted at 1 mm. In a preferred embodiment, the targeted precision is set to about 0.3 mm. Additionally, the reference points 2130 may be prioritized according to local physiological features, such as bone density calculated from the medical images, and define a sampling route. The user may also manually define or adjust the target region 2120 and the amount and positions of the reference points via the user interface 1800.

Preferably, a rough matching process may be performed prior to the Step S2510 to facilitate acquisition of surface information in the subsequent steps. According to an embodiment of the present disclosure, the Step S251 of generating sampling pattern step further includes (S2511) defining a plurality of rough reference points in the target region on the virtual anatomical model; (S2512) prompting the user to generate rough spatial data by sampling a plurality of rough sampling points on the subject corresponding to the reference points; and (S2513) assigning the rough spatial data into the virtual anatomical model.

In Step S2511 of the embodiment, the rough reference points may be defined by the computer system 1700 according to, for example, an imaging processing algorithm. Alternatively, the rough reference points may be defined manually by the user via the user interface 1800. As exemplified in FIG. 10, the rough reference points 2215a-f are preferably located close to the radiopaque spots corresponding to the fiducial marker 1610. The amount of the rough reference points may vary, and is preferably one to ten, or more preferably four to six. After the rough reference points are defined, the user is prompted to sample a plurality of rough sampling points on or around the marker patch 1600 attached to the subject corresponding to the rough reference points 2215a-f using a probe installed on the instrument 1200. The rough sampling points may be sampled one at a time by allowing the computer system to manipulate the kinematic state of the manipulator so that the tip of the probe to contact the rough sampling points or continuously by allowing the tip of the probe to slide along a predefined rough sampling route formed by the rough sampling points. In the embodiment, a spatial marker frame 1550 is attached to the instrument 1200 to allow tracking of the coordinates of the probe by the spatial sensor system 1500 during sampling and thus obtaining rough spatial data 2255 corresponding to the rough reference points 2215a-f. Thereafter, the rough spatial data is assigned into the virtual anatomical model in Step S2512, thereby preliminarily matching the virtual anatomical model into the coordinate system recorded in the computer system 1700.

Alternatively, the rough matching process may be performed automatically by the surgery assistive system 1000. For example, in an embodiment of the present disclosure in which fiducial markers 1610 readable by the spatial sensor system 1510 and radiopaque to the medical imager 1910 are placed on the marker patch 1600 on the subject, the Step S251 of generating sampling pattern step further includes a step of: assigning the rough spatial data of the fiducial markers on the subject into the virtual anatomical model.

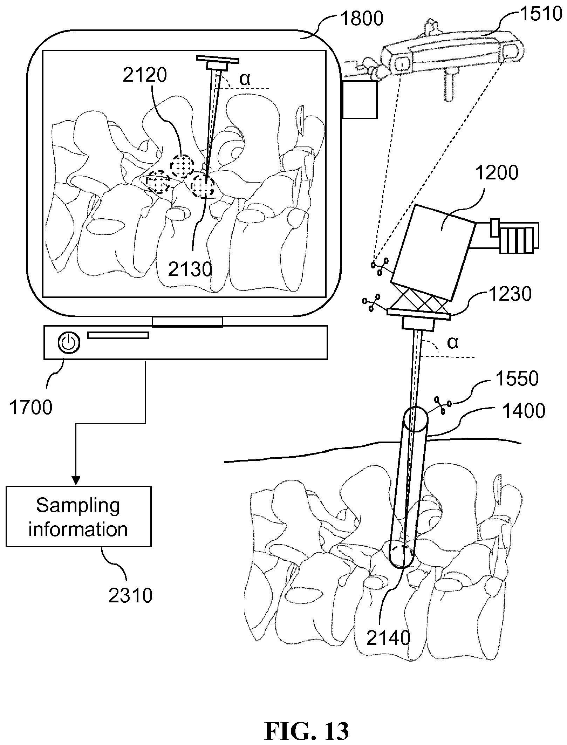

Referring to FIG. 13, together with FIG. 11. After the sampling pattern is generated in Step S251, the user is prompted to generate sampling information 2310 by sampling a plurality of sampling points 2140 on the subject corresponding to the reference points 2130 using a registration probe installed on the instrument 1200, as in Step S252. The sampling points may be sampled one at a time by allowing the computer system to manipulate the kinematic state of the manipulator so that the tip of the probe to contact the sampling points or continuously by allowing the tip of the probe to slide along a predefined sampling route formed by the sampling points. In the embodiment, the sampling information may include coordinates of the sampling points and angles α of contact of the probe at the sampling points as detected by the spatial sensor 1510 and parameters associated with the contacts, such as force sustained by the probe, output power of the actuator, and duration of steady contact.

In the embodiment, the force sustained by the probe including normal force (i.e., the forces parallel to the direction of the probe) and lateral force (i.e., the forces perpendicular to the direction of the probe) is detectable by the force sensor 1235 on the instrument 1200. The angle α of contact may be defined as the angle of the probe in respect to the horizontal plane. The duration of steady contact may be defined as the duration of time that the position of the spatial marker frame 1550 on the platform 1230 of the instrument 1200 remains substantially unchanged or that the acceleration, velocity, displacement, angular velocity and/or angular acceleration detected by the inertial measurement unit of the instrument 1200 remains substantially zero. Additionally, a trocar 1400 may be utilized to guide the probe during surface sampling.

After all of sampling points 2140 on the subject corresponding to the reference points 2130 are sampled in step S2521, the computer system 1700 designates or defines the sampling information as surface information of the sampling points, as in Step S2521. Referring again to FIG. 9. After the surface information is obtained in Step S151, the surface information is assigned into the virtual anatomical model generated in Step S152, thereby registering the virtual anatomical model into the coordinate system established by the spatial sensor system 1500. In the embodiment, an algorithm is employed to identify a surface region in the virtual anatomical model that fits the obtained surface information the most. The algorithm may be iterative closets point (ICP), coherent point drift (CPD) or any algorithm that minimizes the difference between two clouds of points and matches two sets of data.

In a more efficient embodiment, after a predefined amount of sampling points 2140 are sampled by the user in step S252, the computer system 1700 may assign the sampled sampling points 2140 into the virtual anatomical model while the user continues to sample the following sampling points. The results of the assignment would be displayed on the user interface 1800, so that the user can determine whether the sampled sampling points are already sufficient for registering the virtual anatomical model into the coordinate system and stop sampling the remaining sampling points accordingly.

In some embodiments of the present disclosure, the obtained surface information may be utilized to refine or improve the resolution of the virtual anatomical model generated from the medical images in Step S110, by for example updating existing surface features in the virtual anatomical model or adding new surface features to the virtual anatomical model. The surface information may also be used to update the surgical plan generated in Step S141. Alternatively, the surface information and the refined virtual anatomical model may together be utilized to update the surgical plan to ensure higher accuracy of the surgical plan.

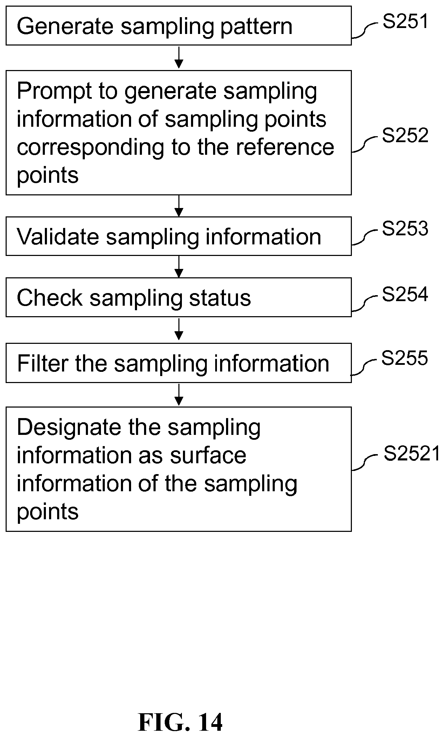

Referring to FIG. 14. According to another embodiment of the present disclosure, the step S 151 of obtaining surface information of the sampling points includes the steps of (S251) generating sampling pattern; (S252) prompting the user to generate sampling information of the sampling points; (S253) validating a current piece of the sampling information; (S254) checking sampling status; (S255) filtering the sampling information and (S2521) designating the sampling information as surface information of the sampling points.

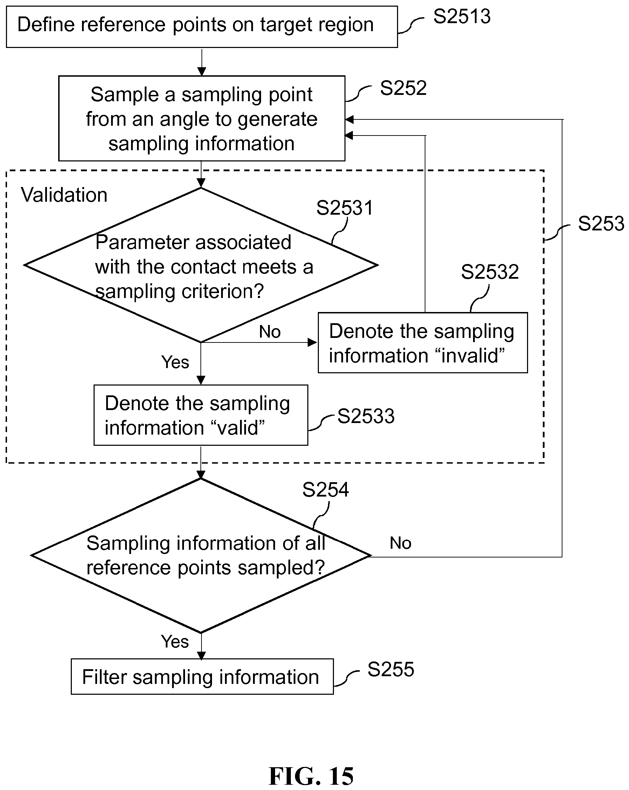

Specifically, as illustrated in FIG. 15, after the sampling information is generated in Step S252, the computer system 1700 validates the sampling information by examining the parameters according to at least one predefined sampling criterion, as in Step S253. The predefined sampling criterion may include, but is not limited to, the normal force sustained by the probe being equal to or stronger than a normal force threshold value, the lateral force sustained by the probe being equal to or weaker than a lateral force threshold value, the torque sustained by the probe being equal to or smaller than a torque threshold value, the output power of the actuator being equal to or higher than a power threshold value, and the duration of steady contact between the probe and the contacted surface being equal to or longer than a time threshold. In some embodiments, the normal force threshold value may be 5 Newton (N), the lateral force threshold value may be 0.5 N, or the torque threshold value may be 0.05 (milli-Newton×meter) mNm; the power threshold value may be 0.3 Amp; and the time threshold may be 1 second.

Referring to FIG. 15. According to an embodiment of the present disclosure, the step S253 of validating a current piece of the sampling information includes the steps of: (S2531) determining if the parameters associated with a contact included in the current piece of sampling information meets at least one of the predefined sampling criterion; (S2532) denoting the current piece of sampling information a first note if the parameters meet the sampling criterion or (S2533) denoting the current piece of sampling information a second note if the parameters do not meet the sampling criterion; and (S252) generating sampling information again if the current piece of sampling information is denoted the second note or (S254) checking sampling status if the current piece of sampling information is denoted the first note.

In the exemplary embodiment as depicted in FIG. 15, after a piece of sampling information is generated by the user contacting a sampling point 2140 on the subject corresponding to a reference point 2130 in the virtual anatomical model 2110 from an angle in Step S252, the computer system 1700 determines in Step S2531 if the parameter included in the piece of sampling information meets at least one of the sampling criterion. If the parameter does not meet the sampling criterion, the computer system 1700 would denote the sampling information "invalid" (S2532) and prompt the user via the user interface 1800 to contact the same sampling point 2140 again from a different angle (S252). In some embodiments, the computer system 1700 may skip a sampling point and prompt the user to contact the next one when sampling of that sampling point fails (e.g., the parameter does not meet the sampling criterion) consecutively for a predefined amount of times.

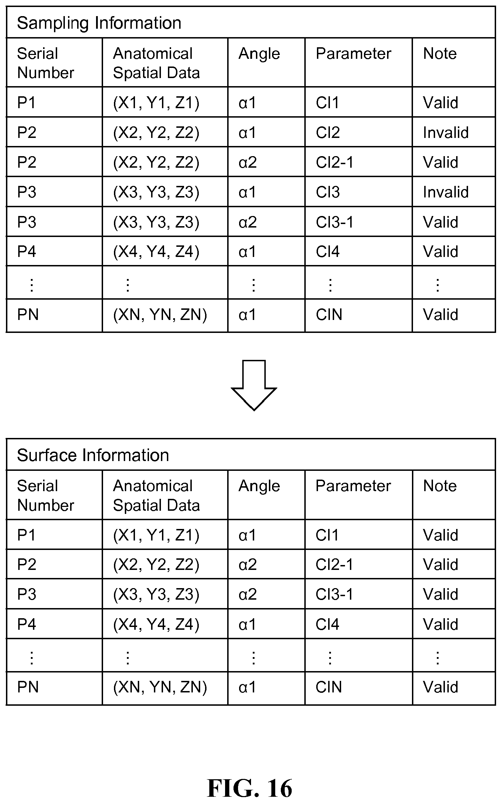

Alternatively, if the parameter meets the sampling criterion, the computer system 1700 would denote the piece of sampling information the first note (e.g., "valid" or "correct") (S2533) and proceed to checking the current sampling status (S254). If the sampling information of all of the sampling points corresponding to the reference points 2130 defined in Step S2513 have not been sampled, the computer system 1700 would prompt the user via the user interface 1800 to contact a next sampling point on the subject according to the predefined sampling route. The computer system 1700 repeats the validation process until all of the sampling points are correctly sampled. After all of the sampling information are sampled, the computer system 1700 proceeds to step S255 and filters the acquired sampling information by screening the invalid ones out from the valid ones. Thereafter, as exemplified in FIG. 16, step S2521 is performed to designate the filtered sampling information as surface information, therefore obtaining a list of surface information of the sampling points.

In a more efficient embodiment, Step S254 may further determine whether to prompt the user to sample the next sampling point on the predefined sampling route according to key surface features associated with the sampled sampling points. The key surface features may include, but are not limited to, peak and valley on the surface as determined according to the detected direction and intensity of force sustained by the probe during sampling. If the computer system 1700 determines not to proceed as originally planned, the computer system 1700 may identify a new sampling point by, for example, deciding a direction that potentially includes one or more of the key surface features according to the parameter in the last sampling information and defining a sampling point in the direction as the next sampling point. Alternatively, the computer system 1700 may redefine the reference points or the sampling route and start sampling according the newly defined sampling route.

In sum, according to a comparative example to the present disclosure, the surgery assistive system provides an accurate and efficient method for registration. The method defines a target region on a surface of a subject and a set of reference points distributed in the target region that covers a plurality of surface features of the surface, and validates the mechanical contacts between the probe of the surgical instrument and the surface, therefore effectively improving the accuracy and precision of computer-assisted surgeries.

## Claims

1. A surgery assistive system (1000), comprising:
an instrument (1100) comprising a tool (1250), a parallel manipulator (1210) connected to the tool, a force sensor (1235) for detecting at least one of force and torque sustained by the tool, wherein the force sensor is connected between the tool and the manipulator;
a spatial sensor system (1500) for detecting spatial information of the tool; and
a computer system (1700) electrically connected to the instrument, the spatial sensor system, and a user interface (1800), for manipulating a kinematic state of the manipulator according to the spatial information of the tool as detected by the spatial sensor system,
wherein the computer system comprises a non-transitory computer readable medium storing a program for obtaining surface information, the program is executable by at least one processor of the computer system and comprises instructions that, when executed by the processor, causes the surgery assistive system to perform actions comprising steps of:
(S1) defining, by the computer system, a target region and a plurality of reference points in the target region on a virtual anatomical model of a subject;
(S2) prompting, via the user interface, a user to generate sampling information by using the instrument to sample a plurality of sampling points on the subject corresponding to the reference points one at a time by allowing the computer system to manipulate the kinematic state of the manipulator so that a tip of the tool of the instrument is configured to contact the sampling points or continuously by allowing the tip of the tool to slide along a predefined sampling route formed by the sampling points, and determining whether to prompt the user to sample the next sampling point on the predefined sampling route according to key surface features associated with the sampled sampling points, the key surface features including peak and valley on the surface as determined according to the detected direction and/or intensity of force sustained by the tool during sampling, and, if the computer system determines not to proceed as originally planned, identifying a new sampling point by:
deciding a direction that potentially includes one or more of the key surface features, the key surface features being peak and valley on the surface as determined according to the detected direction and/or intensity of force sustained by the tool during sampling, according to the parameter in the last sampling information and defining a sampling point in the direction as the next sampling point; or
redefining the reference points or the sampling route and start sampling according the newly defined sampling route,
wherein each piece of the sampling information comprises a coordinate of one of the sampling points, an angle of a contact of the tool at the one of the sampling points as detected by the spatial sensor system, and parameters associated with the contact; and
(S3) designating, by the computer system, the sampling information as surface information of the sampling points, and assigning the surface information into the virtual anatomical model, thereby registering the virtual anatomical model into a coordinate system established by the spatial sensor system.

2. The system according to claim 1, wherein the instrument comprises at least one handle (1284) for allowing a user to hold onto and maneuver the instrument.

3. The system according to claim 1 or 2, wherein the force sensor is disposed in the tool.

4. The system according to any of claims 1 to 3, wherein the parameters associated with the contact comprise the force sustained by the tool.

5. The system according to any of claims 1 to 4, wherein the parameters associated with the contact comprise the torque sustained by the tool.

6. The system according to any of claims 1 to 5, the parameters associated with the contact comprise an output power of a plurality of actuators (1240) of the manipulator.

7. The system according to any of claims 1 to 6, the parameters associated with the contact comprise a duration of steady contact between the tool and one of the sampling points.

## Patentansprüche

1. Operationsunterstützungssystem (1000), umfassend:
ein Instrument (1100), das ein Werkzeug (1250), einen Parallelmanipulator (1210), der mit dem Werkzeug verbunden ist, und einen Kraftsensor (1235) zum Detektieren einer Kraft und/oder eines Drehmoments, die von dem Werkzeug unterstützt werden, umfasst, wobei der Kraftsensor zwischen Werkzeug und Manipulator geschaltet ist;
ein räumliches Sensorsystem (1500) zum Detektieren räumlicher Informationen des Werkzeugs; und
ein Computersystem (1700), das elektrisch mit dem Instrument, dem räumlichen Sensorsystem und einer Benutzerschnittstelle (1800) verbunden ist, um einen kinematischen Zustand des Manipulators gemäß den räumlichen Informationen des Werkzeugs, wie sie vom räumlichen Sensorsystem detektiert werden, zu manipulieren,
wobei das Computersystem ein nicht-transitorisches computerlesbares Medium umfasst, das ein Programm zum Erhalten von Oberflächeninformationen speichert, wobei das Programm durch mindestens einen Prozessor des Computersystems ausführbar ist und Anweisungen umfasst, die, wenn sie durch den Prozessor ausgeführt werden, bewirken, dass das Operationsunterstützungssystem Aktionen durchführt, die die folgenden Schritte umfassen:
(S1) Definieren, durch das Computersystem, einer Zielregion und mehrerer Referenzpunkte in der Zielregion auf einem virtuellen anatomischen Modell eines Probanden;
(S2) Auffordern eines Benutzers über die Benutzerschnittstelle, Probenahmeinformationen zu erzeugen, indem das Instrument verwendet wird, um mehrere Probenahmepunkte auf dem Probanden, die den Referenzpunkten entsprechen, einzeln nacheinander zu beproben, indem dem Computersystem erlaubt wird, den kinematischen Zustand des Manipulators so zu manipulieren, dass eine Spitze des Werkzeugs des Instruments dafür ausgelegt ist, die Probenahmepunkte zu berühren, oder kontinuierlich zu beproben, indem zugelassen wird, dass die Spitze des Werkzeugs entlang einer vordefinierten Probenahmestrecke gleitet, die durch die Probenahmepunkte gebildet wird, und Bestimmen, ob der Benutzer aufgefordert werden soll, den nächsten Probenahmepunkt auf der vordefinierten Probenahmestrecke gemäß den Schlüsseloberflächenmerkmalen, die mit den beprobten Probenahmepunkten verknüpft sind, zu beproben, wobei die Schlüsseloberflächenmerkmale, Spitzen und Täler auf der Oberfläche beinhalten, die gemäß der detektierten Richtung und/oder Intensität der vom Werkzeug während der Probenahme unterstützten Kraft bestimmt werden, und, falls das Computersystem bestimmt, nicht wie ursprünglich geplant vorzugehen, Identifizieren eines neuen Probenahmepunkts durch:
Bestimmen einer Richtung, die potenziell eines oder mehrere der Schlüsseloberflächenmerkmale aufweist, wobei die Schlüsseloberflächenmerkmale Spitzen und Täler auf der Oberfläche sind, die gemäß der detektierten Richtung und/oder der Intensität der von dem Werkzeug während der Probenahme unterstützten Kraft bestimmt werden, gemäß dem Parameter in der letzten Probenahmeinformation, und Definieren eines Probenahmepunkts in der Richtung als nächsten Probenahmepunkt; oder
Neudefinieren der Bezugspunkte oder der Probenahmestrecke und Starten der Probenahme gemäß der neu definierten Probenahmestrecke,
wobei jede Probenahmeinformation eine Koordinate eines der Probenahmepunkte, einen Kontaktwinkel des Werkzeugs an dem einen der Probenahmepunkte, wie er von dem räumlichen Sensorsystem detektiert wird, und mit dem Kontakt verknüpfte Parameter umfasst; und
(S3) Bezeichnen, durch das Computersystem, der Probenahmeinformationen als Oberflächeninformationen der Probenahmepunkte und Zuordnen der Oberflächeninformationen in das virtuelle anatomische Modell, wodurch das virtuelle anatomische Modell in ein durch das räumliche Sensorsystem erstelltes Koordinatensystem registriert wird.

2. System gemäß Anspruch 1, wobei das Instrument mindestens einen Griff (1284) umfasst, der es einem Benutzer ermöglicht, das Instrument festzuhalten und zu manövrieren.

3. System gemäß Anspruch 1 oder 2, wobei der Kraftsensor im Werkzeug angeordnet ist.

4. System gemäß einem der Ansprüche 1 bis 3, wobei die mit dem Kontakt verknüpften Parameter die vom Werkzeug unterstützte Kraft umfassen.

5. System gemäß einem der Ansprüche 1 bis 4, wobei die mit dem Kontakt verknüpften Parameter das vom Werkzeug unterstützte Drehmoment umfassen.

6. System gemäß einem der Ansprüche 1 bis 5, wobei die mit dem Kontakt verknüpften Parameter eine Ausgangsleistung mehrerer Aktuatoren (1240) des Manipulators umfassen.

7. System gemäß einem der Ansprüche 1 bis 6, wobei die mit dem Kontakt verknüpften Parameter eine Dauer des ständigen Kontakts zwischen dem Werkzeug und einem der Probenahmepunkte umfassen.

## Revendications

1. Système d'assistance chirurgicale (1000), comprenant :
un instrument (1100) comprenant un outil (1250), un manipulateur parallèle (1210) relié à l'outil, un capteur de force (1235) pour détecter au moins l'un de la force et du couple subis par l'outil, le capteur de force étant connecté entre l'outil et le manipulateur ;
un système de capteur spatial (1500) pour détecter les informations spatiales de l'outil ; et
un système informatique (1700) connecté électriquement à l'instrument, au système de capteur spatial et à une interface utilisateur (1800), pour manipuler un état cinématique du manipulateur en fonction des informations spatiales de l'outil détectées par le système de capteur spatial,
dans lequel le système informatique comprend un support non transitoire lisible par ordinateur stockant un programme pour l'obtention d'informations de surface, le programme étant exécutable par au moins un processeur du système informatique et comprenant des instructions qui,
lorsqu'elles sont exécutées par le processeur, amènent le système d'assistance chirurgicale à effectuer des actions comprenant les étapes suivantes :
(S1) définir, par le système informatique, une région cible et une pluralité de points de référence dans la région cible sur un modèle anatomique virtuel d'un sujet ;
(S2) inviter, par l'intermédiaire de l'interface utilisateur, un utilisateur à générer des informations d'échantillonnage en utilisant l'instrument pour échantillonner une pluralité de points d'échantillonnage sur le sujet correspondant aux points de référence, un à la fois, en permettant au système informatique de manipuler l'état cinématique du manipulateur de sorte qu'une pointe de l'outil de l'instrument soit configurée pour entrer en contact avec les points d'échantillonnage ou de manière continus en permettant à la pointe de l'outil de glisser le long d'un chemin d'échantillonnage prédéfini formé par les points d'échantillonnage, et en déterminant s'il faut inviter l'utilisateur à échantillonner le point d'échantillonnage suivant sur le chemin d'échantillonnage prédéfini en fonction des caractéristiques de surface clés associées aux points d'échantillonnage échantillonnés, les caractéristiques de surface clés comprenant des pics et des vallées sur la surface tels que déterminés en fonction de la direction et/ou de l'intensité détectées de la force subies par l'outil pendant l'échantillonnage et, si le système informatique détermine qu'il ne faut pas procéder comme prévu à l'origine, en identifiant un nouveau point d'échantillonnage en :
déterminant une direction qui inclut potentiellement une ou plusieurs des caractéristiques clés de la surface, les caractéristiques clés de la surface étant des pics et des vallées sur la surface tels que déterminés en fonction de la direction et/ou de l'intensité détectées de la force subies par l'outil pendant l'échantillonnage, en fonction du paramètre dans les dernières informations d'échantillonnage et en définissant un point d'échantillonnage dans la direction en tant que point d'échantillonnage suivant ; ou
en redéfinissant les points de référence ou le chemin d'échantillonnage et en commençant l'échantillonnage selon le chemin d'échantillonnage nouvellement défini,
dans lequel chaque élément des informations d'échantillonnage comprend une coordonnée de l'un des points d'échantillonnage, un angle de contact de l'outil au niveau de l'un des points d'échantillonnage tel que détecté par le système de capteur spatial, et des paramètres associés au contact ; et
(S3) désigner, par le système informatique, les informations d'échantillonnage en tant qu'informations de surface des points d'échantillonnage, et assigner les informations de surface dans le modèle anatomique virtuel, enregistrant ainsi le modèle anatomique virtuel dans un système de coordonnées établi par le système de capteur spatial.

2. Système selon la revendication 1, dans lequel l'instrument comprend au moins une poignée (1284) permettant à un utilisateur de tenir et de manœuvrer l'instrument.

3. Système selon la revendication 1 ou la revendication 2, dans lequel le capteur de force est disposé dans l'outil.

4. Système selon l'une quelconque des revendications 1 à 3, dans lequel les paramètres associés au contact comprennent la force subie par l'outil.

5. Système selon l'une quelconque des revendications 1 à 4, dans lequel les paramètres associés au contact comprennent le couple subi par l'outil.

6. Système selon l'une quelconque des revendications 1 à 5, dans lequel les paramètres associés au contact comprennent une puissance de sortie d'une pluralité d'actionneurs (1240) du manipulateur.

7. Système selon l'une quelconque des revendications 1 à 6, dans lequel les paramètres associés au contact comprennent une durée de contact stable entre l'outil et l'un des points d'échantillonnage.
